# EUROPEAN PATENT APPLICATION

(11) **EP 3 366 198 A1**
(43) Date of publication of application: **29.08.2018**
(21) Application number: 18150560.3
(22) Date of filing: 08.01.2018
(51) Int. Cl.: A61B 5/00, A61B 5/055

(54) **METHODS AND APPARATUSES FOR MONITORING CONDITION OF OBJECT IN MEDICAL IMAGING**

(30) Priority: 28.02.2017 KR 20170026666; 25.09.2017 KR 20170123651
(71) Applicant: Samsung Electronics Co., Ltd., Gyeonggi-do 16677 (KR)
(72) Inventor: Lee, Jun-ki, Seoul (KR)
(74) Representative: Gulde & Partner

(57) **Abstract**

A method for monitoring a condition of an object includes obtaining a condition signal of the object in an operation of imaging the object to obtain a medical image of the object; determining whether the condition of the object is an abnormal condition based on the condition signal, by using a model that is set to determine the abnormal condition of the object; and in response to determining that the condition of the object is the abnormal condition, performing an operation corresponding to the abnormal condition of the object, wherein the model is generated by learning data obtained in relation to the object by using an artificial intelligence (AI) algorithm.

## Description

### CROSS-REFERENCE TO RELATED APPLICATION

This application claims priority from Korean Patent Application No. 10-2017-0026666, filed on February 28, 2017, and Korean Patent Application No. 10-2017-0123651, filed on September 25, 2017, in the Korean Intellectual Property Office, the disclosures of which are incorporated herein in their entireties by reference.

### BACKGROUND

### 1. Field

Methods and apparatuses consistent with the exemplary embodiments relate to methods and apparatuses for monitoring a condition of an object (e.g., a patient) in medical imaging, and more particularly, to patient monitoring methods and patient monitoring apparatuses, wherein a patient's condition is monitored during medical imaging and a suitable operation is performed based on the patient's condition.

Also, methods and apparatuses consistent with the exemplary embodiments relate to an artificial intelligence (Al) system and application thereof, which simulate functions of a human brain, such as recognition and decision-making, by using a machine learning algorithm.

### 2. Description of the Related Art

A magnetic resonance imaging (MRI) system is an apparatus for noninvasively obtaining a magnetic resonance image including information about biological tissues of a patient by using a magnetic field. The MRI system obtains information about an inner body by using resonance of hydrogen (H) atoms in water forming most of a human body.

Obtaining of a medical image using such an MRI system is widely used as a method for diagnosing and treating a patient. However, in order to generate a signal and obtain an image, the MRI system continuously applies electromagnetic waves to the human body, and radio frequency (RF) energy used at this time may be absorbed by and heat human tissue. If the heating is not controlled, the cardiovascular system and the nervous system of the patient may be disabled, and the heating may burn skin severely.

Accordingly, in order to prevent an accident such as a burn, which may occur during an MRI, a user manipulating the MRI system needs to continuously monitor a patient's condition. In particular, in cases where the patient suffers from heart disease, has an advanced disease, the patient is anesthetized, or the patient is a child, an expert needs to observe the patient continuously and closely.

Meanwhile, recently, an artificial intelligence (AI) system realizing intelligence of a human is used in various fields. The Al system is a system in which a machine self-learns, determines, and becomes intelligent, unlike an existing rule-based smart system. The more the Al system is used, a recognition rate is increased and a user's preference is more accurately understood, and thus the existing rule-based smart system is gradually replaced by a deep-learning-based Al system.

An Al technology includes machine learning and element technologies using the machine learning.

The machine learning is an algorithm technology that self-classifies and learns features of input data. An element technology is a technology for simulating functions of a human brain, such as recognition and determination, by using a machine learning algorithm, such as deep-learning, and includes a technical field of linguistic understanding, visual understanding, inference/prediction, knowledge representation, or operation control.

Various fields to which the Al technology is applied are as follows. Verbal understanding is a technology of recognizing languages/characters of people and applying/processing the languages/characters, and includes natural language processing, machine translation, a dialog system, questions and answers, or voice recognition/synthesis. Visual understanding is a technology of recognizing an object like in human vision, and includes object recognition, object tracing, image search, person recognition, scene understanding, space understanding, or image improvement. Inference/prediction is a technology of logically inferring and predicting information by determining the information, and includes knowledge/probability-based inference, optimization prediction, preference-based plans, or recommendation. Knowledge representation is a technology of automating experience information to knowledge data, and includes knowledge construction (data generation/classification) or knowledge management (data application). Operation control is a technology of controlling automatic driving of a vehicle or movement of a robot, and includes movement control (navigation, collision, driving) or manipulation control (action control).

In order for a user to continuously observe a patient's condition, a general (or related art) medical imaging system may include a display displaying a bio-signal of the patient in real time. The user may check various bio-signals of the patient provided through the display, and determine whether the patient's condition is within a normal range based on the bio-signals. According to the general medical imaging system, the user is required to depend on personal judgment in selecting a bio-signal to be considered first to determine abnormality of the patient and determining whether the bio-signal is within a normal range.

Also, according to the general medical imaging system, in order to monitor the patient's condition during medical imaging, the user is required to have relevant expertise or participation of a relevant expert is necessary, and the user needs to simultaneously monitor various bio-signals per patient.

According to the general medical imaging system, it may be difficult for the user to determine importance and normal ranges of the bio-signals of the patient solely based on personal judgment. Also, it is possible the user make mistakes while determining abnormality of the patient and simultaneously considering the bio-signals of the patient, which change in real time.

### SUMMARY

One or more exemplary embodiments provide a patient monitoring apparatus, wherein a patient's condition is automatically monitored to avoid issues (or problems) that may arise when the patient's condition is monitored based on subjective judgment of a user.

One or more exemplary embodiments provide a patient monitoring apparatus, wherein a patient's condition is learned based on machine learning, and an optimum action is performed based on the patient's condition.

Additional aspects will be set forth in part in the description which follows and, in part, will be apparent from the description, or may be learned by practice of the presented embodiments.

According to an aspect of an embodiment, provided is a method for monitoring a condition of an object, including: obtaining a condition signal of the object in an operation of imaging the object to obtain a medical image of the object; determining whether the condition of the object is an abnormal condition based on the condition signal, by using a model that is set to determine the abnormal condition of the object; and in response to determining that the condition of the object is the abnormal condition, performing an operation corresponding to the abnormal condition of the object, wherein the model is generated by learning data obtained in relation to the object by using an artificial intelligence (Al) algorithm.

According to an aspect of another embodiment, provided is an apparatus for monitoring a condition of an object, including: a model generated by learning data obtained in relation to the object by using an artificial intelligence (Al) algorithm, and set to determine an abnormal condition of the object; and at least one processor configured to determine, based on a condition signal of the object obtained in an operation of imaging the object to obtain a medical image of the object, whether the condition of the object is the abnormal condition, by using the model which is set to determine the abnormal condition of the object, and in response to determining that the condition of the object is the abnormal condition, perform an operation corresponding to the abnormal condition of the object.

According to an aspect of another embodiment, provided is a non-transitory computer program product including a computer-readable recording medium that stores at least one program to execute a method including: obtaining, from at least one sensor, a condition signal of an object in an operation of imaging the object to obtain a medical image of the object; determining whether a condition of the object is an abnormal condition based on the condition signal, by using a model which is set to determine the abnormal condition of the object; and in response to determining that the condition of the object is the abnormal condition, performing an operation corresponding to the abnormal condition of the object, wherein the model is generated by learning data obtained in relation to the object by using an artificial intelligence (Al) algorithm.

According to an aspect of another embodiment, provided is system for monitoring a condition of an object, including: an apparatus configured to determine, based on a condition signal of the object obtained in an operation of imaging the object to obtain a medical image of the object, whether the condition of the object is an abnormal condition by using a model which is set to determine the abnormal condition of the object, and in response to determining that the condition of the object to be the abnormal condition, perform an operation corresponding to the abnormal condition of the object; and a medical image obtaining apparatus configured to perform an operation of obtaining the medical image of the object in response to determining that the condition of the object is a normal condition, wherein the model is generated by learning data obtained in relation to the object by using an artificial intelligence (AI) algorithm.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a diagram for describing a general medical imaging system;
FIG. 2 is a block diagram of a patient monitoring apparatus according to an embodiment;
FIG. 3 is a block diagram of a patient monitoring apparatus according to another embodiment;
FIG. 4 is a block diagram of a processor according to an embodiment;
FIG. 5 is a flowchart of a patient monitoring method performed by a patient monitoring apparatus, according to an embodiment;
FIG. 6 is a flowchart of a patient monitoring method performed by a patient monitoring apparatus, according to another embodiment;
FIG. 7 is a block diagram of a structure of a magnetic resonance imaging (MRI) system, according to an embodiment;
FIG. 8 is a block diagram of a learning unit and a determining unit, according to an embodiment; and
FIG. 9 is a flowchart of a system using a determination model, according to an embodiment.

### DETAILED DESCRIPTION

Advantages and features of one or more exemplary embodiments of the disclosure and methods of accomplishing the same may be understood more readily by reference to the following detailed description of the embodiments and the accompanying drawings. In this regard, the exemplary embodiments may have different forms and should not be construed as being limited to the descriptions set forth herein. Rather, these embodiments are provided so that this disclosure will be thorough and complete and will fully convey the concept of the exemplary embodiments to one of ordinary skill in the art, and the disclosure will only be defined by the appended claims. In drawings, well-known functions or constructions are not illustrated for clarity of description, and like reference numerals denote like elements.

Reference will now be made in detail to embodiments, examples of which are illustrated in the accompanying drawings, wherein like reference numerals refer to like elements throughout. In this regard, the exemplary embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

One or more embodiments of the disclosure may be described in terms of functional block components and various processing steps. Some or all of such functional blocks may be realized by any number of hardware and/or software components configured to perform the specified functions. For example, the functional blocks of the disclosure may be realized by one or more microprocessors or by circuit components for certain functions. Also, for example, the functional blocks of the disclosure may be realized in various programming or scripting languages. The functional blocks may be implemented in algorithms that execute on one or more processors. Furthermore, the disclosure could employ any number of conventional techniques for electronics configuration, signal processing and/or control, data processing, and the like.

Furthermore, connecting lines or connectors between elements shown in drawings are intended to represent exemplary functional relationships and/or physical or logical couplings between the elements. It should be noted that many alternative or additional functional relationships, physical connections or logical connections may be present in a practical device.

In the specification, an "object" may be organic or inorganic. Also, the object may denote all or a part of a body of a patient, and may include an organ, such as the liver, the heart, the womb, the brain, a breast, or the abdomen, or an embryo, or may include a cross-section of the body.

In the specification, a "user" may be a medical expert, such as a medical doctor, a nurse, a medical laboratory technologist, a sonographer, or a medical image expert, but is not limited thereto. Also, in the specification, a "medical image" may include a sonogram, a magnetic resonance (MR) image, an x-ray image, a computerized tomography (CT) image, a positron emission tomography (PET) image, or a model image in which an object is modeled, but is not limited thereto.

In the specification, "'medical image data" denotes a data set for generating a medical image, and image data may include volume data and 2-dimensional (2D) image data.

Reference will now be made in detail to embodiments, examples of which are illustrated in the accompanying drawings. In this regard, the exemplary embodiments may have different forms and should not be construed as being limited to the descriptions set forth herein.

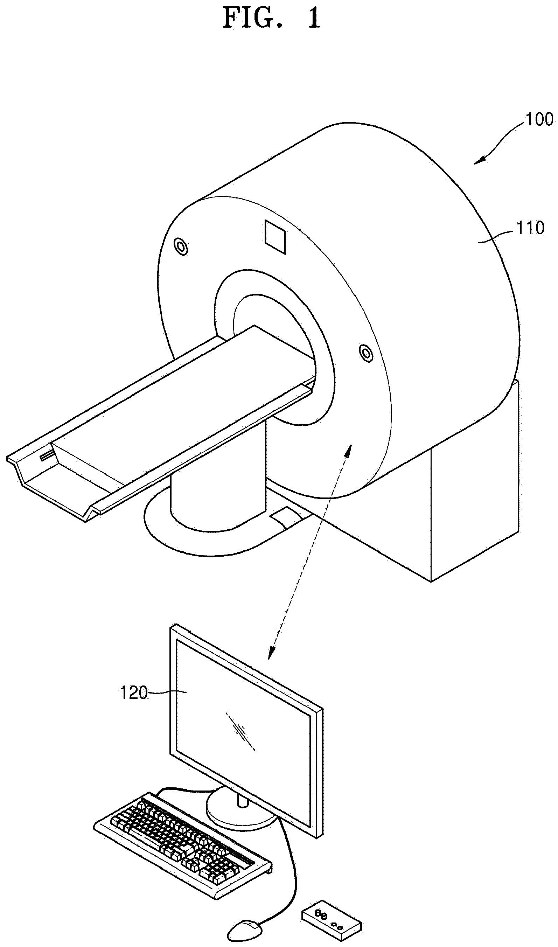

FIG. 1 is a diagram for describing a general medical imaging system 100. For example, the medical imaging system 100 of FIG. 1 may be a magnetic resonance imaging (MRI) system or a CT imaging system, but is not limited thereto.

As shown in FIG. 1, the medical imaging system 100 may include a medical image obtaining apparatus 110 and a medical image display apparatus 120.

The medical image obtaining apparatus 110 may include a table to support an object, and a gantry to irradiate a certain signal (for example, an x-ray, an MR signal, or ultrasonic waves) towards the object. The medical image obtaining apparatus 110 may receive a signal generated by the object. The medical image obtaining apparatus 110 may obtain a medical image of the object based on the received signal.

The medical image display apparatus 120 may receive the medical image obtained by the medical image obtaining apparatus 110, and display the medical image on a screen. Also, the medical image display apparatus 120 may control the medical image obtaining apparatus 110.

FIG. 1 illustrates the medical image display apparatus 120 separate from the medical image obtaining apparatus 110, but embodiments are not limited thereto. The medical image display apparatus 120 may be included in the medical image obtaining apparatus 110 or connected to the medical image obtaining apparatus 110. The medical image display apparatus 120 may include a console controlling the medical image obtaining apparatus 110. The medical image obtaining apparatus 110 and the medical image display apparatus 120 may be connected to each other through a wired/wireless network.

The medical image display apparatus 120 may display not only the medical image of the object, but also medical image obtaining information related to the obtaining of the medical image. For example, the medical image obtaining information may include information about execution phases to be processed by the medical imaging system 100 so as to provide the medical image obtained from the object to a user. The medical imaging system 100 may, through a user interface (UI) related to the execution phases, receive a set value related to the execution phases, receive information related to the object, or output a state of the object or the medical imaging system 100.

Also, the medical image display apparatus 120 may display information about the state of the object. For example, the medical image display apparatus 120 may display a bio-signal obtained from the object.

The user of the medical imaging system 100 may check various bio-signals provided through the medical image display apparatus 120 and determine whether a condition of a patient is within a normal range based on the bio-signals, so as to continuously monitor the condition of the patient. According to the general medical imaging system 100, the user is required to depend on personal judgment in selecting a bio-signal to be considered first and determining the normal bio-signal range, so as to determine an abnormal condition of the patient.

Also, according to the general medical imaging system 100, in order to monitor a patient's condition during medical imaging, the user needs to have relevant expertise or participation of a relevant expert is necessary, and the user needs to simultaneously monitor several bio-signals per patient.

Accordingly, exemplary embodiments provide a patient monitoring apparatus in which a patient's condition is automatically monitored so as to avoid issues (or problems) generated when the patient's condition is monitored based on subjective judgment of a user. Also, exemplary embodiments provide a patient monitoring apparatus, in which a patient's condition is learned based on machine learning, and an optimum action is performed based on the patient's condition.

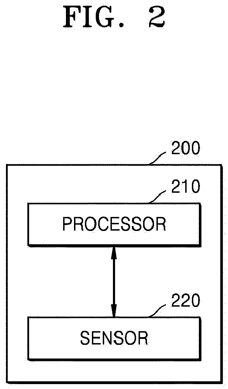

FIG. 2 is a block diagram of a patient monitoring apparatus 200, according to an embodiment.

The patient monitoring apparatus 200 according to an embodiment may be included, for example, in a medical imaging system for obtaining and displaying a medical image, such as an MRI system or a CT imaging system. However, embodiments are not limited thereto, and the patient monitoring apparatus 200 may be an apparatus for processing or displaying a medical image obtained from a separate apparatus, without having to directly obtain a medical image from an object. Alternatively, the patient monitoring apparatus 200 may be included in a medical image obtaining apparatus or a medical image display apparatus. Alternatively, the patient monitoring apparatus 200 may be operated as a separate setup box connected to the medical image obtaining apparatus or the medical image displaying apparatus wirelessly or via wires. Alternatively, the patient monitoring apparatus 200 may be included in at least one server connected to the medical image obtaining apparatus or the medical image display apparatus wirelessly. Examples of the at least one server may include a cloud server sharing at least one of a plurality of processors and a plurality of memories for certain purposes. The patient monitoring apparatus 200 according to an embodiment may include a processor 210 and a sensor 220. In FIG. 2, only one processor 210 is illustrated, but there may be two or more processors 210, according to an embodiment.

According to an embodiment, the processor 210 may control overall operations of the patient monitoring apparatus 200. The processor 210 according to an embodiment may generate a model that is set to determine a normal condition or an abnormal condition (or unusual condition or irregular condition) of a patient by learning data obtained in relation to the patient. The processor 210 may receive a condition signal of the patient from at least one sensor 220 relating to information of operation of imaging the patient to obtain a medical image of the patient.

The information of operation of imaging the patient may include at least one of a time in which the processor 210 prepares to image the patient, a time in which the processor 210 images the patient, a time in which the processor 210 obtains medical image data of the patient, a time in which the processor 210 generates a medical image by using the medical image data, a time in which the processor 210 obtains the generated medical image, and a time in which the processor 210 displays the obtained medical image. Also, the information of operation of imaging the patient may include, when the patient is imaged a plurality of times, a time interval between each of the plurality of imaging. The time interval may include a time in which the patient or the user prepares for next imaging, a time in which an image protocol is adjusted for the next imaging, and a time in which the medical image data is generated.

The processor 210 may determine whether the condition of the patient is a normal condition or an abnormal condition (or unusual condition or irregular condition) based on the condition signal received from the at least one sensor 220, by using the generated model.

The data obtained in relation to the patient may include at least one medical record of the patient, current health of the patient, medication information of the patient, and an image protocol used to obtain the medical image of the patient.

The processor 210 may generate the model by learning at least one of data obtained in relation to other patients and a pre-imaging condition signal of the patient, together with the data obtained in relation to the patient. The data obtained in relation to the other patients may include at least one medical record of the other patients, medication information of the other patients, image protocols used to obtain medical images of the other patients, condition signals of the other patients before or while the medical images of the other patients are obtained, and abnormality of the other patients while the medical images of the other patients are obtained. The data obtained in relation to the other patients may be empirical data collected in the past during medical imaging of a plurality of patients. The pre-imaging condition signal of the patient may include information about a condition of the patient obtained before the medical image of the patient is obtained.

The processor 210 may obtain at least one portion of the data obtained in relation to the patient and the data obtained in relation to the other patients from a memory (not shown) embedded in the patient monitoring apparatus 200 or an external memory (not shown) connected to the patient monitoring apparatus 200. Alternatively, the processor 210 may obtain at least one portion of the data obtained in relation to the patient and the data obtained in relation to the other patients from a server (not shown).

The processor 210 according to an embodiment may generate the model by performing machine learning on the data obtained in relation to the patient. The processor 210 may generate the model by learning at least one portion of the data obtained in relation to the other patients and the pre-imaging condition signal of the patient, together with the data obtained in relation to the patient.

The machine learning may denote that an electronic device, which includes a processor, learns based on empirical data, performs prediction, and self-improves performance. The electronic device performing machine learning may build a dynamic model to derive prediction, determination, or estimation based on input data, instead of performing strictly defined static program commands or performing an operation based on a uniformly defined function. An example of the dynamic model may include a model that is set to determine an abnormal condition or a normal condition of a patient, as will be described later.

For example, the processor 210 may use, as a method of an Al algorithm or a machine learning algorithm, at least one of an artificial neural network, a Bayesian network, a support vector machine, and a decision tree. The decision tree is an analysis method of performing classification and prediction by schematizing decision rules in a tree structure. The Bayesian network is a method of using a model representing, in a graph structure, a probabilistic relation between a plurality of variables, such as conditional independence. The support vector machine is a method of using a model of supervised learning for pattern recognition and data analysis. The artificial neural network is obtained by modeling a connection relationship between neurons and an operation principle of biological neurons, and is a method of using a model in which a plurality of neurons, such as nodes or processing elements, are connected in a layer structure.

The processor 210 according to an embodiment may generate a model by generating a plurality of nodes included in the model and assigning a weight that is adjustable based on learned data to each of the plurality of nodes. The plurality of nodes may be connected to each other to form a network. Each of the plurality of nodes may include at least one of at least one function and at least one parameter.

The model generated by the processor 210 may determine whether the condition of the patient is a normal condition or an abnormal condition based on input data. The processor 210 may obtain a result of determining whether the condition of the patient is the normal condition or the abnormal condition by transmitting the condition signal of the patient to the plurality of nodes. The processor 210 may determine whether the condition of the patient is the normal condition or the abnormal condition based on the result. The processor 210 may determine whether the condition of the patient is the normal condition or the abnormal condition based on the condition signal of the patient and a protocol performed to obtain the medical image of the patient.

The model generated by the processor 210 is a dynamic model using weights that are adjusted based on learned data, and thus different results may be derived according to patients or protocols of patients used to capture medical images of the patients. Also, since the model generated by the processor 210 may self-improve performance based on learned data, determination results having higher accuracy may be derived as data of a greater number of patients is learned.

When the condition of the patient is determined to be a normal condition, the processor 210 may control a medical image obtaining apparatus connected to the processor 210 to continuously perform an operation of obtaining the medical image.

On the other hand, when the condition of the patient is determined to be an abnormal condition, the processor 210 may perform an operation suitable for the condition of the patient. For example, the processor 210 may control the medical image obtaining apparatus or a medical image display apparatus based on the condition of the patient.

For example, when the condition of the patient is determined to be the abnormal condition, the processor 210 may, based on the condition of the patient, change a protocol for obtaining the medical image, stop the operation of obtaining the medical image, restart the operation of obtaining the medical image, or control the medical image obtaining apparatus such that the patient is removed from the medical image obtaining apparatus. For example, when the condition of the patient is determined to be the abnormal condition, the processor 210 may attempt to communicate with the patient or notify the user that the condition of the patient is the abnormal condition, based on the condition of the patient.

When the condition of the patient is determined to be the abnormal condition, the processor 210 according to an embodiment may notify the user that the condition of the patient is the abnormal condition. When a user input is received, the processor 210 may control the medical image obtaining apparatus to continuously perform the operation for obtaining the medical image. For example, despite the determination of the processor 210, when a user input indicating that the condition of the patient is the normal condition or a user input for ignoring the determination of the processor 210 is received, the processor 210 may control the medical image obtaining apparatus to continuously perform the operation for obtaining the medical image.

The patient monitoring apparatus 200 according to an embodiment may include the at least one sensor 220. The sensor 220 may detect the condition of the patient in the operation of imaging the patient, so as to obtain the medical image of the patient.

The sensor 220 may obtain the condition signal by detecting the condition of the patient while the medical image of the patient is obtained or before the medical image of the patient is obtained. Also, the sensor 220 may obtain the condition signal by detecting the condition of the patient during at least one of a time in which the processor 210 prepares to image the patient, a time in which the processor 210 images the patient, a time in which the processor 210 obtains medical image data of the patient, and a time in which the processor 210 generates a medical image by using the medical image data. Alternatively, when the patient is imaged a plurality of times, the sensor 220 may obtain the condition signal by detecting the condition of the patient during a time interval between each of the plurality of imaging times.

The condition signal including information about the condition of the patient may include a bio-signal of the patient. For example, the sensor 220 may obtain, as the condition signal, information about at least one of an image signal of the patient, a voice signal of the patient, an electrocardiogram, a heart rate, blood pressure, oxygen saturation, a respiration state, a respiration rate, a temperature of the patient, and a temperature around the patient.

The sensor 220 may be disposed near the patient, attached onto the skin of the patient, or disposed on an external surface of the medical image obtaining apparatus.

According to some embodiments, the condition signal including the information about the condition of the patient may be obtained by analyzing the medical image of the patient. In this case, the medical image of the patient may be a pre-captured medical image or a medical image obtained in the operation of imaging the patient. For example, in an MRI system, a low-quality abdomen image of the patient may be obtained in real time. In this case, a respiration state or a respiration rate of the patient may be obtained as the condition signal of the patient based on analysis of the low-quality abdomen image.

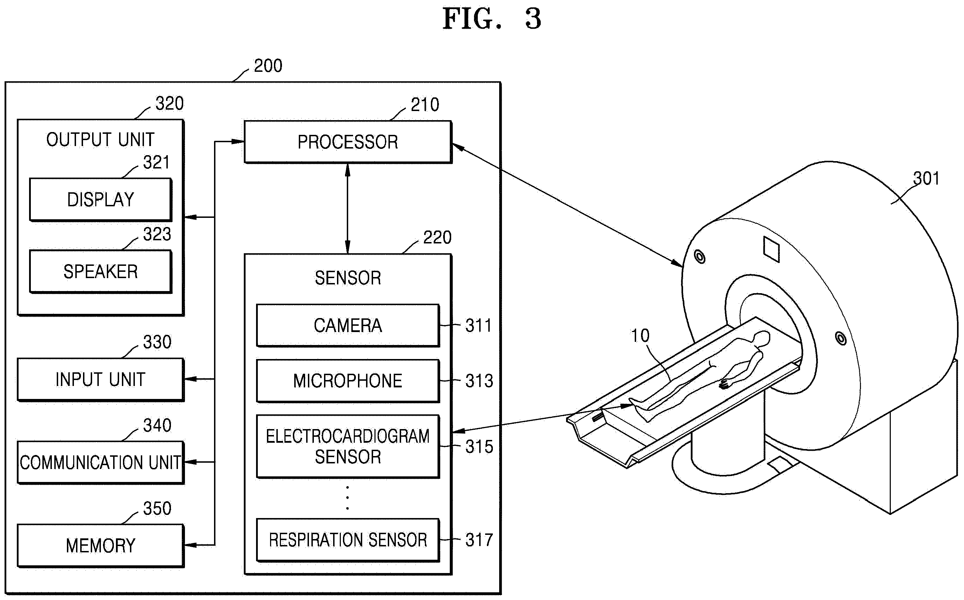

FIG. 3 is a block diagram of the patient monitoring apparatus 200, according to another embodiment.

The patient monitoring apparatus 200 according to an embodiment is not limited to that shown in FIG. 2. The patient monitoring apparatus 200 may include more components than those shown in FIG. 2. For example, as shown in FIG. 3, the patient monitoring apparatus 200 according to an embodiment may further include at least one of an output unit 320, an input unit 330, a communication unit 340, and a memory 350. The patient monitoring apparatus 200 may be included in a medical image obtaining apparatus 301 obtaining a medical image of a patient 10, or connected to the medical image obtaining apparatus 301 wirelessly or via wires.

According to an embodiment, at least one sensor 220 included in the patient monitoring apparatus 200 may include a camera 311, a microphone 313, an electrocardiogram sensor 315, and a respiration sensor 317. However, an embodiment is not limited thereto, and the sensor 220 may further include a blood pressure sensor, an oxygen saturation sensor, and a thermometer. Types of sensors included in the patient monitoring apparatus 200 may vary according to a patient to be monitored.

The camera 311 may obtain an image signal of the patient 10. The camera 311 may monitor at least one of a condition and movement of the patient 10. The processor 210 may determine a level of pain felt by the patient 10 based on the image signal of the patient 10 received from the camera 311. The processor 210 may determine shaking or sweating of the patient 10 from the image signal received from the camera 311. Also, the camera 311 may further include an infrared camera for measuring a skin temperature of the patient 10.

The microphone 313 may obtain a voice signal of the patient 10. The processor 210 may recognize the condition of the patient 10 from the voice signal received through the microphone 313. Also, the microphone 313 may form an intercom system together with a speaker 323. The processor 210 may recognize voice of the patient 10 through the microphone 313 included in the intercom system and provide feedback through the speaker 323. The electrocardiogram sensor 315 may obtain an electrocardiogram of the patient 10. The respiration sensor 317 may detect a respiration state or a respiration rate of the patient 10.

The output unit 320 according to an embodiment may output a result of monitoring the patient 10. The output unit 320 may output at least one of a condition signal of the patient 10, information about whether the condition of the patient 10 is a normal condition or an abnormal condition, a medical image obtained with respect to the patient 10, medical image obtaining information related to obtaining of the medical image, information about execution phases to be processed by a medical imaging system to obtain the medical image, an image protocol, a UI for receiving a user input, and a UI for controlling the medical image obtaining apparatus 301 connected to the patient monitoring apparatus 200. For example, the output unit 320 may include a display 321 for outputting an audio signal or a video signal, and the speaker 323.

The output unit 320 according to an embodiment may perform an operation suitable for the condition of the patient 10. For example, when the condition of the patient 10 is determined to be an abnormal condition, the display 321 may display a notification to the user indicating that the condition of the patient 10 is the abnormal condition, display a UI for receiving a user input for changing a protocol for obtaining the medical image, display a UI for receiving a user input for stopping the operation of obtaining the medical image, display a UI for receiving a user input for restarting the operation of obtaining the medical image, or display a UI for receiving a user input for removing the patient 10 from the medical image obtaining apparatus 301. For example, the speaker 323 may perform communication with the patient 10 while being attached near the medical image obtaining apparatus 301 where the patient 10 is located. For example, when the condition of the patient 10 is determined to be the abnormal condition, the speaker 323 may be used to notify the user that the condition of the patient 10 is the abnormal condition.

The input unit 330 according to an embodiment may receive a user input for controlling at least one of the patient monitoring apparatus 200 and the medical image obtaining apparatus 301 connected to the patient monitoring apparatus 200. Examples of the user input may include an input of manipulating a button, a keypad, a mouse, a track ball, a jog switch, or a knob, an input of touching a touch pad or a touch screen, a voice input, a motion input, and a bio-information input (for example, iris recognition or fingerprint recognition), but are not limited thereto.

The communication unit 340 according to an embodiment may include one or more components enabling communication with an external device, and for example, may include at least one of a short-range communication module, a wired communication module, and a wireless communication module.

The communication unit 340 may receive a control signal and data from the external device and transmit the received control signal to the processor 210 such that the processor 210 controls the patient monitoring apparatus 200 according to the received control signal. Alternatively, the processor 210 may transmit a control signal to the external device through the communication unit 340 so as to control a controller of the external device according to the control signal. For example, the medical image obtaining apparatus 301 may perform a medical image obtaining operation suitable for the condition of the patient 10, based on a control signal received through the communication unit 340. Also, the communication unit 340 may receive the condition signal from the sensor 220, or may obtain at least one of data related to the patient 10 and data related to other patients from an external memory or server.

The memory 350 may store various types of data or programs for driving and controlling the patient monitoring apparatus 200. Also, the memory 350 may store at least one of the data related to the patient 10 and the data related to the other patients. Also, the memory 350 may store medical image data obtained from the patient, and the medical image.

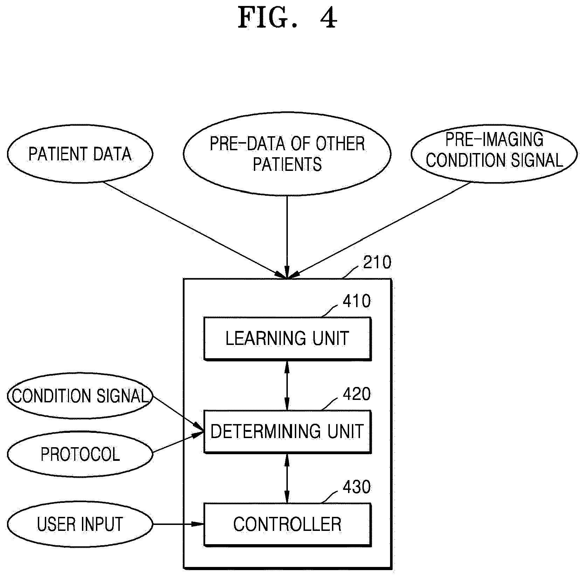

FIG. 4 is a block diagram of the processor 210 included in the patient monitoring apparatus 200, according to an embodiment.

The processor 210 according to an embodiment may include a learning unit 410, a determining unit 420, and a controller 430.

The processor 210 may control the patient monitoring apparatus 200 based on machine learning according to an embodiment, such that abnormality of a condition of a patient is automatically detected in real time during medical imaging. The processor 210 may process condition signals obtained from a plurality of sensors, and control a medical image obtaining apparatus or a peripheral device based on a result of the processing.

The learning unit 410 according to an embodiment may learn to distinguish the normal condition and the abnormal condition of the patent. The learning unit 410 may generate a model determining whether the condition of the patient is the normal condition or the abnormal condition by learning pre-data related to other patients and data related to the patient whose medical image is to be obtained currently. The learning unit 410 may generate the model by learning data obtained from other patients, medical records of a patient to be currently examined, a current condition of the patient, medication of the patient, and a medical imaging protocol to be used. Also, the learning unit 410 may increase accuracy of the generated model by additionally learning a pre-imaging condition signal of the patient obtained in the normal condition of the patient before medical imaging of the patient.

The determining unit 420 may determine the condition of the patient based on the condition signal of the patient received from the sensor 220. When the learning unit 410 generates the model, a medical image obtaining apparatus starts to perform medical imaging of the patient. The determining unit 420 may continuously determine the condition of the patient during medical imaging by applying to the model the condition signal of the patient obtained from the sensor 220 in real time during medical imaging. The determining unit 420 may determine the condition of the patient by further considering the medical imaging protocol currently performed by the medical image obtaining apparatus, together with the condition signal of the patient.

The controller 430 may control the medical image obtaining apparatus or the peripheral device, or notify the condition of the patient to the user based on the condition of the patient determined by the determining unit 420. When it is determined that the condition of the patient is the abnormal condition, the controller 430 may perform an operation suitable for the condition of the patient. For example, the controller 430 may control the medical image obtaining apparatus to change the medical imaging protocol, stop the medical imaging temporarily, restart the medical imaging, or immediately remove the patient from a scanner. Alternatively, the controller 430 may notify the user of the abnormal condition of the patient.

Hereinafter, a patient monitoring method according to an embodiment will be described. Each operation of the patient monitoring method may be performed by each component of the patient monitoring apparatus 200 described above, and overlapping descriptions are not provided again.

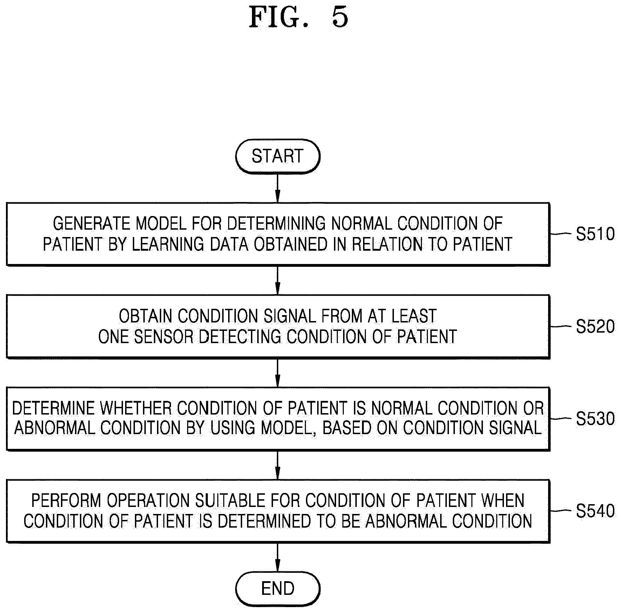

FIG. 5 is a flowchart of a patient monitoring method performed by the patient monitoring apparatus 200, according to an embodiment. Hereinafter, for convenience of description, the patient monitoring method of FIG. 5 is performed by the patient monitoring apparatus 200. However, embodiments are not limited thereto, and the patient monitoring method of FIG. 5 may be performed by an electronic apparatus included in at least one of a medical imaging system, a medical image obtaining apparatus, and a medical image display apparatus, or may be connected thereto wirelessly or via wires.

In operation S510, the patient monitoring apparatus 200 according to an embodiment may generate a model for determining a normal condition of a patient by learning data obtained in relation to the patient. The patient monitoring apparatus 200 may generate the model by performing machine learning on the obtained data.

The patient monitoring apparatus 200 according to an embodiment may generate the model by learning the data obtained in relation to the patient including medical records of the patient or data obtained in relation to other patients.

Before obtaining a medical image of the patient, the patient monitoring apparatus 200 according to an embodiment may obtain a pre-imaging condition signal from at least one sensor. The patient monitoring apparatus 200 may generate the model by learning the data obtained in relation to the patient and further learning the pre-imaging condition signal.

For example, the model generated by the patient monitoring apparatus 200 may include a plurality of nodes forming a network by being connected to each other. For example, the patient monitoring apparatus 200 may generate the plurality of nodes included in the model. The patient monitoring apparatus 200 may assign a weight that is adjustable based on the learned data to each of the plurality of nodes. Each of the plurality of nodes may include at least one function. The patient monitoring apparatus 200 may transmit the condition signal of the patient obtained from the patient during medical imaging to the plurality of nodes included in the mode to determine whether a condition of the patient is a normal condition or an abnormal condition.

In operation S520, the patient monitoring apparatus 200 according to an embodiment may obtain the condition signal from the at least one sensor detecting the condition of the patient while obtaining the medical image of the patient. For example, the condition of the patient may include information about at least one of an image signal of the patient, a voice signal of the patient, an electrocardiogram, a heart rate, blood pressure, oxygen saturation, a respiration state, a respiration rate, a temperature of the patient, and a temperature around the patient.

In operation S530, the patient monitoring apparatus 200 according to an embodiment may determine whether the condition of the patient is a normal condition or an abnormal condition by using the model, based on the condition signal of the patient. By applying the condition signal to the model, the patient monitoring apparatus 200 may determine whether the condition of the patient is a normal condition or an abnormal condition.

The patient monitoring apparatus 200 according to an embodiment may determine whether the condition of the patient is a normal condition or an abnormal condition by using the model, by further considering a medical imaging protocol performed to obtain the medical image, together with the condition signal.

In operation S540, when the condition of the patient is determined to be the abnormal condition, the patient monitoring apparatus 200 according to an embodiment may perform an operation suitable for the condition of the patient. For example, the operation suitable for the condition of the patient may include at least one of changing the medical imaging protocol for obtaining the medical image, stopping the operation of obtaining the medical image, restarting the operation of obtaining the medical image, attempting communication with the patient, notifying the abnormal condition of the patient, and removing the patient from the medical image obtaining apparatus.

When the condition of the patient is determined to be the abnormal condition, the patient monitoring apparatus 200 according to an embodiment may notify a user that the condition of the patient is the abnormal condition. Upon receiving a user input, the patient monitoring apparatus 200 may continue the operation of obtaining the medical image.

On the other hand, when the condition of the patient is determined to be the normal condition, the patient monitoring apparatus 200 according to an embodiment may continue the operation of obtaining the medical image.

The patient monitoring apparatus 200 may monitor the condition of the patient during the medical imaging, and when the patient is abnormal, suggest a corresponding procedure based on the condition of the patient or automatically perform the operation suitable for the condition of the patient. Accordingly, according to an embodiment, mistakes that the user that may make in relation to recognizing or responding to the abnormal condition of the patient may be reduced.

For example, when a medical image is captured with respect to a patient with an advanced disease, a patient suffering from a cardiovascular disease, or a patient under anesthesia, the patient monitoring apparatus 200 according to an embodiment may generate a model by considering (or primarily considering) a vital sign of the patient. The patient monitoring apparatus 200 may determine whether the condition of the patient is a normal condition or an abnormal condition by using the generated model. The patient monitoring apparatus 200 may generate a model in which a vital sign of the patient is more important than other parameters, by learning the data obtained in relation to the patient. In order to generate the model, the patient monitoring apparatus 200 may further learn the normal condition of the patient before the medical imaging. The patient monitoring apparatus 200 may recognize a risk of the patient in an early stage, and perform a corresponding procedure, such as notifying the user or stopping the medical imaging, based on the condition signal of the patient continuously obtained during the medical imaging.

As another example, when a medical image is captured with respect to a patient suffering from a mental illness, such as claustrophobia or depression, the patient monitoring apparatus 200 according to an embodiment may generate a model by considering (or primarily considering) a condition signal indicating whether a mental condition of the patient is stable or unstable. The patient monitoring apparatus 200 may determine whether the condition of the patient is a normal condition or an abnormal condition by using the model. The patient monitoring apparatus 200 may generate a model in which the condition signal indicating whether the mental condition of the patient is stable or unstable is more important than other parameters, by learning the data obtained in relation to the patient.

The patient monitoring apparatus 200 may detect the condition of the patient continuously obtained during the medical imaging, and induce continuous communication with the patient based on the condition of the patient. The patient monitoring apparatus 200 may perform basic conversation, such as asking how the patient feels, based on the detected condition of the patient. The patient monitoring apparatus 200 may determine that it is difficult to obtain the medical image of the patient when the patient moves too much due to physical or mental instability, based on the condition signal of the patient continuously obtained during the medical imaging. When the condition of the patient is determined to be the abnormal condition and it is therefore difficult to continue the medical imaging, the patient monitoring apparatus 200 may temporarily stop the medical imaging or change the medical imaging protocol.

As another example, when a medical image of a patient who has difficulty with communication is obtained, the patient monitoring apparatus 200 according to an embodiment may generate a model by considering (or primarily considering) a vital sign obtained from the patient or pain of the patient. The patient monitoring apparatus 200 may determine whether a condition of the patient is a normal condition or an abnormal condition by using the generated model. The patient monitoring apparatus 200 may determine a pain level of the patient based on a condition signal including information related to shaking or sweating of the patient obtained from the sensor 220. The patient monitoring apparatus 200 may automatically monitor the condition of the patient based on the condition signal continuously obtained during the medical imaging, and notify the user of whether it is possible to continue the medical imaging based on the condition of the patient.

As another example, the patient monitoring apparatus 200 may measure a skin temperature of the patient by using an infrared camera-based temperature sensor. The patient monitoring apparatus 200 may generate a model for determining a normal condition of the patient by learning the data obtained in relation to the patient. In order to generate the model, the patient monitoring apparatus 200 may pre-learn basic information about the medical imaging protocol and equipment, a weight of the patient, and a body type of the patient. The patient monitoring apparatus 200 may determine whether the condition of the patient is dangerous (or alerting) by analyzing an image continuously obtained from the infrared camera during the medical imaging.

When it is determined that the condition of the patient is outside a normal range and thus is dangerous, the patient monitoring apparatus 200 may control the medical image obtaining apparatus to change the medical imaging protocol or stop the medical imaging so as to prevent a burn caused by a specific absorption rate (SAR). Alternatively, when it is determined that the condition of the patient is outside the normal range and thus is dangerous, the patient monitoring apparatus 200 may notify this to the user.

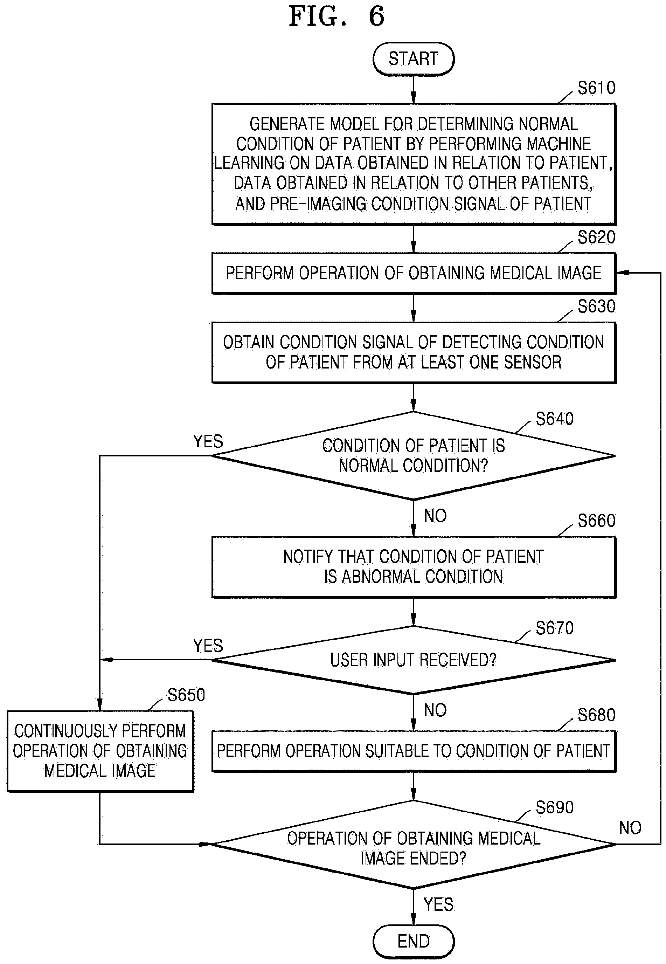

FIG. 6 is a flowchart of a patient monitoring method performed by the patient monitoring apparatus 200, according to another embodiment.

Operation S510 of FIG. 5 may correspond to operation S610 of FIG. 6, operation S520 of FIG. 5 may correspond to operation S630 of FIG. 6, operation S530 of FIG. 5 may correspond to operation S640 of FIG. 6, and operation S540 of FIG. 5 may correspond to operations S660 to S690 of FIG. 6. Descriptions in FIG. 5 may be applied to operations of FIG. 6 corresponding to those of FIG. 5, and thus repetitive descriptions are not provided.

In operation S610, the patient monitoring apparatus 200 according to an embodiment may generate a model for determining a normal condition of a patient by performing machine learning. The patient monitoring apparatus 200 may perform the machine learning on data obtained in relation to the patient, data obtained in relation to other patients, and a pre-imaging condition signal of the patient.

In operation S620, the patient monitoring apparatus 200 according to an embodiment may control a medical image obtaining apparatus to perform an operation for obtaining a medical image. The medical image obtaining apparatus may sequentially perform one or more of a plurality of execution phases to obtain the medical image from the patient.

The plurality of execution phases to obtain the medical image may be determined based on a user input or based on information stored in a memory. When an imaging region of the patient varies, the plurality of execution phases may also vary. Also, even when one imaging region is imaged, the plurality of execution phases may vary when a medical imaging protocol varies.

In operation S630, the patient monitoring apparatus 200 according to an embodiment may obtain a condition signal of detecting the condition of the patient from at least one sensor. The patient monitoring apparatus 200 may obtain the condition signal periodically.

In operation S640, the patient monitoring apparatus 200 according to an embodiment may determine whether the condition of the patient is a normal condition or an abnormal condition based on the model generated in operation S610. The patient monitoring apparatus 200 may determine whether the condition of the patient is a normal condition or an abnormal condition by applying the condition signal to the model.

In operation S650, when the condition of the patient is determined to be the normal condition, the patient monitoring apparatus 200 according to an embodiment may control the medical image obtaining apparatus to continuously perform an operation for obtaining the medical image.

In operation S660, when the condition of the patient is determined to be the abnormal condition, the patient monitoring apparatus 200 according to an embodiment may notify the user that the condition of the patient is the abnormal condition.

In operation S670, the patient monitoring apparatus 200 according to an embodiment may determine whether a user input to ignore the determination of the patient monitoring apparatus 200 is received. When the user input to ignore the determination of the patient monitoring apparatus 200 is received, the patient monitoring apparatus 200 may control the medical image obtaining apparatus to continuously perform the operation for obtaining the medical image in operation S650.

In operation S680, when the user input to ignore the determination of the patient monitoring apparatus 200 is not received, the patient monitoring apparatus 200 may perform an operation suitable for the condition of the patient. For example, the patient monitoring apparatus 200 may control the medical image obtaining apparatus to change the medical imaging protocol or stop the medical imaging.

In operation S690, the patient monitoring apparatus 200 according to an embodiment may determine whether the operation of obtaining the medical image has ended as the plurality of execution phases to obtain the medical image are all performed. When the operation has not ended, the patient monitoring apparatus 200 may perform operation S620 to control the medical image obtaining apparatus to continue the operation of obtaining the medical image.

In FIG. 6, the operation of obtaining the medical image is continuously performed if the user input is received, and the operation suitable for the condition of the patient is performed if the user input is not received. However, an embodiment is not limited thereto.

For example, when a user input for performing the operation suitable for the condition of the patient according to the determination of the patient monitoring apparatus 200 is received, the patient monitoring apparatus 200 according to an embodiment may perform the operation suitable for the condition of the patient. On the other hand, when the user input for performing the operation suitable for the condition of the patient according to the determination of the patient monitoring apparatus 200 is not received, the patient monitoring apparatus 200 may control the medical image obtaining apparatus to continuously perform the operation of obtaining the medical image.

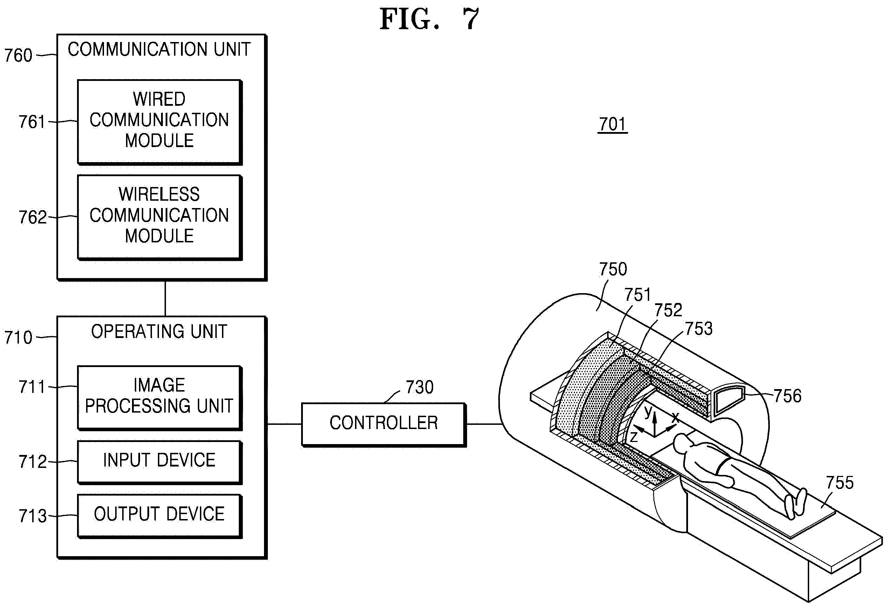

FIG. 7 is a block diagram of a structure of a magnetic resonance imaging (MRI) system 701, according to an embodiment.

The patient monitoring apparatus 200 according to an embodiment may be included in a medical image obtaining system or connected to the medical image obtaining system wirelessly or via wires.

Examples of the medical image obtaining system may include an MRI system, a CT system, and an X-ray system, but are not limited thereto.

For example, the MRI system acquires an MR signal and reconstructs the acquired MR signal into an image. The MR signal denotes a radio frequency (RF) signal emitted from the object.

In the MRI system, a main magnet generates a static magnetic field to align a magnetic dipole moment of a specific atomic nucleus of the object placed in the static magnetic field along a direction of the static magnetic field. A gradient coil may generate a gradient magnetic field by applying a gradient signal to a static magnetic field, and induce resonance frequencies differently according to each region of the object.

An RF coil may emit an RF signal to match a resonance frequency of a region of the object of which an image is to be acquired. Furthermore, when gradient magnetic fields are applied, the RF coil may receive MR signals having different resonance frequencies emitted from a plurality of regions of the object. Through this process, the MRI system may obtain an image from an MR signal by using an image reconstruction technique.

Referring to FIG. 7, the MRI system 701 may include an operating unit 710, a controller 730, and a scanner 750. The controller 730 may be independently separate from the operating unit 710 and the scanner 750. Furthermore, the controller 730 may be separated into a plurality of sub-components and incorporated into the operating unit 710 and the scanner 750 in the MRI system 701. Operations of the components in the MRI system 701 will now be described in detail.

The scanner 750 may be formed to have a cylindrical shape (e.g., a shape of a bore) having an empty inner space into which an object may be positioned. A static magnetic field and a gradient magnetic field are generated in the inner space of the scanner 750, and an RF signal is emitted toward the inner space.

The scanner 750 may include a static magnetic field generator 751, a gradient magnetic field generator 752, an RF coil unit 753, a table 755, and a display 756. The static magnetic field generator 751 generates a static magnetic field for aligning magnetic dipole moments of atomic nuclei of the object in a direction of the static magnetic field. The static magnetic field generator 751 may be formed as a permanent magnet or superconducting magnet using a cooling coil.

The gradient magnetic field generator 752 is connected to the controller 730 and generates a gradient magnetic field by applying a gradient to a static magnetic field in response to a control signal received from the controller 730. The gradient magnetic field generator 752 includes X, Y, and Z coils for generating gradient magnetic fields in X-, Y-, and Z-axis directions crossing each other at right angles and generates a gradient signal according to a position of a region being imaged so as to differently induce resonance frequencies according to regions of the object.

The RF coil unit 753 connected to the controller 730 may emit an RF signal toward the object in response to a control signal received from the controller 730 and receive an MR signal emitted from the object. In detail, the RF coil unit 753 may transmit, toward atomic nuclei of the object having precessional motion, an RF signal having the same frequency as that of the precessional motion, stop transmitting the RF signal, and then receive an MR signal emitted from the object.

The RF coil unit 753 may be formed as a transmitting RF coil for generating an electromagnetic wave having an RF corresponding to the type of an atomic nucleus, a receiving RF coil for receiving an electromagnetic wave emitted from an atomic nucleus, or a transmitting/receiving RF coil serving both functions of the transmitting RF coil and the receiving RF coil. Furthermore, in addition to the RF coil unit 753, a separate coil may be attached to the object. Examples of the separate coil may include a head coil, a spine coil, a torso coil, and a knee coil according to a region being imaged or a region to which the separate coil is attached.

The display 756 may be disposed outside and/or inside the scanner 750. The display 756 is also controlled by the controller 730 to provide a user or the object with information related to medical imaging.

Furthermore, the scanner 750 may include an object monitoring information acquisition unit (not shown) configured to acquire and transmit monitoring information about a state of the object. For example, the object monitoring information acquisition unit may acquire monitoring information related to the object from a camera (not shown) for capturing images of movement or a position of the object, a respiration measurer (not shown) for measuring respiration of the object, an electrocardiogram (ECG) measurer for measuring electrical activity of the heart, or a temperature measurer for measuring a temperature of the object, and transmit the acquired monitoring information to the controller 730. The controller 730 may in turn control an operation of the scanner 750 based on the monitoring information. Operations of the controller 730 will now be described in more detail.

The controller 730 may control overall operations of the scanner 750.

The controller 730 may control a sequence of signals formed in the scanner 750. The controller 730 may control the gradient magnetic field generator 752 and the RF coil unit 753 according to a pulse sequence received from the operating unit 710 or a designed pulse sequence.

A pulse sequence may include all pieces of information used to control the gradient magnetic field generator 752 and the RF coil unit 753. For example, the pulse sequence may include information about strength, duration, and application timing of a pulse signal applied to the gradient magnetic field generator 752.

The controller 730 may control a waveform generator (not shown) for generating a gradient wave, e.g., an electrical pulse, according to a pulse sequence and a gradient amplifier (not shown) for amplifying the generated electrical pulse and transmitting the same to the gradient magnetic field generator 752. Thus, the controller 730 may control formation of a gradient magnetic field by the gradient magnetic field generator 752.

Furthermore, the controller 730 may control an operation of the RF coil unit 753. For example, the controller 730 may supply an RF pulse having a resonance frequency to the RF coil unit 730 that emits an RF signal toward the object, and receive an MR signal received by the RF control unit 753. In this case, the controller 730 may adjust emission of an RF signal and reception of an MR signal according to an operating mode by controlling an operation of a switch (e.g., a T/R switch) for adjusting transmitting and receiving directions of the RF signal and the MR signal based on a control signal.

The controller 730 may control movement of the table 755 on which the object is placed. Before MRI is performed, the controller 730 may move the table 755 according to which region of the object is to be imaged.

The controller 730 may also control the display 756. For example, the controller 730 may control an on/off state of the display 756 or a screen to be output on the display 756, according to a control signal.

The controller 730 may be formed in a memory (not shown) storing data in a form of an algorithm or program for controlling operations of the components in the MRI system 701, and a processor (not shown) that performs the above-described operations using the data stored in the memory. In this case, the memory and the processor may be implemented as separate chips. Alternatively, the memory and the processor may be incorporated into a single chip.

The operating unit 710 may control overall operations of the MRI system 701 and include an image processing unit 711, an input device 712, and an output device 713.

The image processing unit 711 may control the memory to store an MR signal received from the controller 730, and generate image data with respect to the object from the stored MR signal by applying an image reconstruction technique by using an image processor.

For example, if a k space (for example, also referred to as a Fourier space or a frequency space) of the memory is filled with digital data to complete k-space data, the image processing unit 711 may reconstruct image data from the k-space data by applying various image reconstruction techniques (e.g., by performing inverse Fourier transform on the k-space data) by using the image processor.

Furthermore, the image processing unit 711 may perform various signal processing operations on MR signals in parallel. For example, the image processing unit 711 may perform signal processing on a plurality of MR signals received via a multi-channel RF coil in parallel so as to convert the plurality MR signals into image data. In addition, the image processing unit 711 may store the image data in the memory, or the controller 730 may store the same in an external server via a communication unit 760, as will be described below.

The input device 712 may receive, from the user, a control command for controlling the overall operations of the MRI system 701. For example, the input device 712 may receive, from the user, object information, parameter information, a scan condition, and information about a pulse sequence. The input device 712 may be a keyboard, a mouse, a track ball, a voice recognizer, a gesture recognizer, a touch screen, or any other input device.

The output device 713 may output image data generated by the image processing unit 711. The output device 713 may also output a user interface (UI) configured such that the user may input a control command related to the MRI system 701. The output device 713 may be formed as a speaker, a printer, a display, or any other output device.

Furthermore, although FIG. 7 shows that the operating unit 710 and the controller 730 are separate components, the operating unit 710 and the controller 730 may be included in a single device as described above. Furthermore, processes respectively performed by the operating unit 710 and the controller 730 may be performed by another component. For example, the image processing unit 711 may convert an MR signal received from the controller 730 into a digital signal, or the controller 730 may directly perform the conversion of the MR signal into the digital signal.

The MRI system 701 may further include a communication unit 760 and be connected to an external device (not shown) such as a server, a medical apparatus, and a portable device (e.g., a smartphone, a tablet personal computer (PC), a wearable device, etc.) via the communication unit 760.

The communication unit 760 may include at least one component that enables communication with an external device. For example, the communication unit 760 may include at least one of a local area communication module (not shown), a wired communication module 761, and a wireless communication module 762.

The communication unit 760 may receive a control signal and data from an external device and transmit the received control signal to the controller 730 so that the controller 730 may control the MRI system 701 according to the received control signal.

Alternatively, by transmitting a control signal to an external device via the communication unit 760, the controller 730 may control the external device according to the control signal.

For example, the external device may process data of the external device according to a control signal received from the controller 730 via the communication unit 760.

A program for controlling the MRI system 701 may be installed on the external device and may include instructions for performing some or all of the operations of the controller 730.

The program may be preinstalled on the external device, or a user of the external device may download the program from a server providing an application for installation. The server providing an application may include a recording medium having the program recorded thereon.

The medical image obtaining system may include a patient monitoring apparatus. In this case, the patient monitoring apparatus and the medical image obtaining apparatus may together be referred to as a patient monitoring system. Alternatively, the patient monitoring apparatus, the medical image obtaining apparatus, and a medical image display apparatus may together be referred to as a patient monitoring system.

The patient monitoring apparatus may determine whether a condition of a patient is an abnormal condition by using a model that is set to determine an abnormal condition of the patient, based on a condition signal of the patient obtained in a situation where the patient is imaged to obtain a medical image of the patient. Here, the model may be generated by learning data obtained in relation to the patient by using an Al algorithm.

When the condition of the patient is determined to be the abnormal condition, the patient monitoring apparatus may perform an operation suitable for the condition of the patient. On the other hand, when the condition of the patient is determined to be a normal condition, the medical image obtaining apparatus may obtain the medical image of the patient.

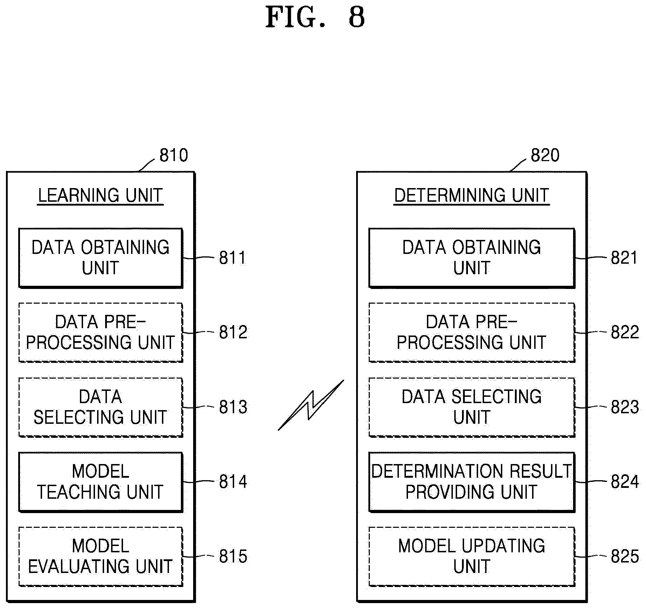

FIG. 8 is a block diagram of a learning unit 810 and a determining unit 820, according to an embodiment.

In FIG. 8, the learning unit 810 and the determining unit 820 work in association with each other to learn and determine data.

Referring to FIG. 8, the learning unit 810 may generate a model that is set to determine a normal condition or an abnormal condition of a patient by using data obtained in relation to the patient, and the determining unit 820 may determine a condition of the patient by using the generated model and perform an operation suitable for the condition of the patient.

For example, the learning unit 810 may be a part of a processor of a server, and the determining unit 820 may be a part of a processor of the patient monitoring apparatus 200. In this case, the learning unit 810 and the determining unit 820 may be connected to each other through a communication network. The communication network may include a wired network or a wireless network.

Also, when the learning unit 810 and the determining unit 820 are implemented by at least one processor of one apparatus, the learning unit 810 and the determining unit 820 may be connected to each other through a data line or a bus.

Here, the learning unit 810 and the determining unit 820 being connected to each other through the communication network may include the learning unit 810 and the determining unit 820 being directly connected to each other or being connected to each other through another element (for example, as a third element, at least one of an access point (AP), a hub, a relay, a base station, a router, and a gateway).

The learning unit 810 may generate a determination model determining whether the patient is in a normal condition or an abnormal condition by using learning data. The determination model being generated by the learning unit 810 may mean that the learning unit 810 teaches the determination model or updates the learned taught model.

The determining unit 820 may determine whether the patient is in the normal condition or in the abnormal condition by applying feature data to the determination model. Also, the determining unit 820 may provide a result of the determination to the controller 430 of FIG. 4. The controller 430 may control a medical image obtaining apparatus or a peripheral device, or perform a function of notifying a user of the condition of the patient based on the result of the determination.

In FIG. 8, the learning unit 810 may include a data obtaining unit 811, a data pre-processing unit 812, a data selecting unit 813, a model teaching unit 814, and a model evaluating unit 815. According to an embodiment, the learning unit 810 includes the data obtaining unit 811 and the model teaching unit 814, and may selectively further include or not include at least one of the data pre-processing unit 812, the data selecting unit 813, and the model evaluating unit 815.

The data obtaining unit 811 may obtain data. For example, the data obtaining unit 811 may obtain data related to the patient or data related to other patients from at least one of a server (for example, a medical management server), an external memory, and a medical image obtaining apparatus.

The data pre-processing unit 812 may pre-process the obtained data. For example, the data pre-processing unit 812 may process the obtained data to a pre-set format such that the model teaching unit 814 described below may use the obtained data. For example, when the data related to the patient includes medical records, the data pre-processing unit 812 may process a condition signal of the patient included in the medical records to a pre-set format.

The data selecting unit 813 may select learning data for learning from among the pre-processed data related to the patient. The selected learning data may be provided to the model teaching unit 814. In this case, the data obtained by the data obtaining unit 811 or the data processed by the data pre-processing unit 812 may be provided to the model teaching unit 814 as the learning data.

The data selecting unit 813 may select the learning data according to a pre-set standard. The pre-set standard may be determined by considering at least one of, for example, a data attribute, a data generation time, a data generator, data reliability, a data target, a data generation region, and a data size.

The model teaching unit 814 may teach the determination model by using the learning data. For example, the model teaching unit 814 may teach the determination model via a supervised learning method or an unsupervised learning method so as to generate a model that is set to determine the abnormal condition or normal condition of the patient. The model teaching unit 814 may generate the model as a dynamic model for deriving prediction, determination, or estimation by using the learning data via machine learning described above.

Here, the determination model may be a pre-built model. For example, the determination model may be a model pre-built by using basic learning data (for example, sample data). In this case, the model teaching unit 814 may teach the pre-built model to provide higher performance.

There may be a plurality of pre-built models. In this case, the model teaching unit 814 may determine, as a model to be taught, a data recognition model having a high relation to input learning data and basic learning data.

The model teaching unit 814 may teach the data recognition model by using, for example, a teaching algorithm including error back-propagation or gradient descent. Also, the model teaching unit 814 may teach the determination model via supervised learning that uses, as a criterion, at least some of the learning data (for example, reference information indicating whether the patient is in the normal condition or the abnormal condition).

Here, the learning data may include data related to the patient obtained when the patient is in the normal condition, or data related to the patient obtained when the patient is in the abnormal condition. Also, the learning data may include reference information indicating whether the data related to the patient is obtained when the patient is in the normal condition or the abnormal condition.

The data related to the patient may include a bio-signal of the patient. For example, when the data related to the patient is medical records of the patient, information about the bio-signal of the patient may include at least one of an electrocardiogram, a heart rate, blood pressure, oxygen saturation, a respiration rate, and a temperature of the patient, which are measured during hospitalization of the patient. In this case, the model teaching unit 814 may teach the determination model by using the medical records of the patient and the reference information indicating whether the medical records are obtained when the patient is in the normal condition or the abnormal condition.

Also, the data related to the patient may include a medical image of the patient and a medical imaging protocol of the medical image. In this case, the model teaching unit 814 may teach the determination model by using the medical image, the medical imaging protocol, and reference information. Here, the reference information may include information indicating whether the medical image is captured when the patient is in the normal condition or the abnormal condition.

Also, the data related to the patient may include a pre-imaging condition signal of the patient obtained from at least one sensor. The pre-imaging condition signal may include a pre-imaging condition signal obtained when the patient is in the normal condition or the abnormal condition. In this case, the learning data may include reference information indicating whether the pre-imaging condition signal is obtained when the patient is in the normal condition or the abnormal condition.

As another example, the model teaching unit 814 may teach the data recognition model via unsupervised learning in which a criterion for determining the condition of the patient by using the learning data without particular supervision is found.

In this case, the model teaching unit 814 may use various types of data related to the patient, as the learning data. For example, the learning data may include at least one of the medical records of the patient, a current condition of the patient, and the medical imaging protocol to be used. Also, the model teaching unit 814 may include the data related to the other patients, as the learning data. The data related to the other patients may include, for example, medical records of the other patients, medical images of the other patients, and medical imaging protocols of the medical images of the other patients.

When the data recognition model is taught via unsupervised learning, the model teaching unit 814 may teach a model considering, for example, a range, a distribution form, or a feature of the data related to the patient. For example, the model teaching unit 814 may classify, from among information about condition signals of the patient included in the data related to the patient, a condition signal within a certain range as data related to the patient in the normal condition, and a condition signal outside the certain range as data related to the patient in the abnormal condition. Here, the certain range may be determined via repetitive learning.

Also, the model teaching unit 814 may also teach a model by using the data related to the other patients. For example, the model teaching unit 814 may classify, from among information about condition signals of patients included in the data related to the other patients and the data related to the patient, a condition signal within the certain range as data related to the patient in the normal condition, and a condition signal outside the certain range as data related to the patient in the abnormal condition.

Also, the model teaching unit 814 may find a criterion for determining the condition of the patient by assigning a weight to information having high importance from among the condition signals of the patient included in the data related to the patient. For example, the model teaching unit 814 may teach the determination model such that a high weight is assigned to condition signals related to a vital sign or pain of the patient.

Also, the model teaching unit 814 may teach the determination model such that a high weight is assigned to condition signals highly related to symptoms of the patient in consideration of a disease of the patient. For example, the determination model may be taught such that a high weight is assigned to a condition signal related to a mental condition of the patient who suffers from a mental illness. Alternatively, the determination model may be taught such that a high weight is assigned to an electrocardiogram, a heart rate, and blood pressure of the patient who suffers from heart disease.

According to various embodiments, the model teaching unit 814 may teach the determination model via reinforcement learning that uses feedback about whether classification according to learning is correct. In this case, the feedback may be obtained during the teaching process of the determination model or obtained based on feedback of a user of the patient monitoring apparatus 200 according to a result of determining the condition of the patient.

After teaching the determination model, the model teaching unit 814 may store the data recognition model. In this case, the model teaching unit 814 may store the determination model in a memory inside or outside the patient monitoring apparatus 200. Alternatively, the model teaching unit 814 may store the determination model in a memory of a server connected to the patient monitoring apparatus 200 wirelessly or via wires.

In this case, the memory where the determination model is stored may also store, for example, a command or data related to at least one of other components of the patient monitoring apparatus 200. Also, the memory may store software and/or a program. The program may include, for example, a kernel, middleware, an application programming interface (API) and/or an application program (or "application").

The model evaluating unit 815 may input evaluation data to the determination model, and when a determination result output from the evaluation data does not satisfy a certain standard, the model evaluating unit 815 may enable the model teaching unit 814 to teach the determination model again. In this case, the evaluation data may be pre-set data for evaluating the determination model.

For example, when the number or proportion of pieces of evaluation data of which determination results from among determination results of the determination model using the evaluation data are not accurate is higher than a pre-set threshold value, the model evaluating unit 815 may evaluate that the certain standard is not satisfied. For example, when the certain standard is defined to be a proportion of 2% (for example, when the determination model outputs wrong determination results with respect to 20 or more pieces of evaluation data from among 1000 pieces of evaluation data), the model evaluation unit 815 may determine that the determination model is not suitable.

There may be a plurality of determination models. In this case, the model evaluating unit 815 may evaluate whether each determination model satisfies the certain standard, and determine the determination model that satisfies the certain standard as a final determination model. At this time, when there are a plurality of determination models that satisfy the certain standard, the model evaluating unit 815 may determine a pre-set certain number of determination models as the final determination model in order of highest evaluation score.

At least one of the data obtaining unit 811, the data pre-processing unit 812, the data selecting unit 813, the model teaching unit 814, and the model evaluating unit 815 of the learning unit 810 may be manufactured in at least one hardware chip from and included in an electronic device. For example, at least one of the data obtaining unit 811, the data pre-processing unit 812, the data selecting unit 813, the model teaching unit 814, and the model evaluating unit 815 may be manufactured in an exclusive hardware chip for Al or may be manufactured as part of an existing general-purpose processor (for example, a central processing unit (CPU) or an application processor) or a graphic-exclusive processor (for example, a graphical processing unit (GPU)), and included in any electronic device.

Also, the data obtaining unit 811, the data pre-processing unit 812, the data selecting unit 813, the model teaching unit 814, and the model evaluating unit 815 may be included in one electronic device or on different electronic devices. For example, some of the data obtaining unit 811, the data pre-processing unit 812, the data selecting unit 813, the model teaching unit 814, and the model evaluating unit 815 may be included in the patient monitoring apparatus 200, and the remainder may be included in a server.

Also, at least one of the data obtaining unit 811, the data pre-processing unit 812, the data selecting unit 813, the model teaching unit 814, and the model evaluating unit 815 may be implemented in a software module (or a program module including an instruction). Here, the software module may be stored in a non-transitory computer-readable recording medium. The software module may be provided by an operating system (OS) or a certain application. Alternatively, a part of the software module may be provided by an OS and the remainder of the software module may be provided by a certain application.

The determining unit 820 of FIG. 8 may include a data obtaining unit 821, a data pre-processing unit 822, a data selecting unit 823, a determination result providing unit 824, and a model updating unit 825. According to various embodiments, the determining unit 820 may include the data obtaining unit 821 and the determination result providing unit 824, and further selectively include at least one of the data pre-processing unit 822, the data selecting unit 823, and the model updating unit 825.

The data obtaining unit 821 may obtain data related to a patient who is a current target. For example, the data obtaining unit 821 may obtain the data related to the patient from at least one of a medical image obtaining apparatus and an external apparatus. The data related to the patient may include data based on a condition signal obtained from at least one sensor detecting a condition of the patient, in an operation of imaging the patient. For example, the data related to the patient may include the condition signal or data obtained by processing the condition signal to a pre-set format.

The data pre-processing unit 822 may pre-process the obtained data. For example, the data obtaining unit 821 may process the obtained data to a pre-set format such that the determination result providing unit 824 described later is able to use the obtained data.

The data selecting unit 823 may select feature data that is a determination target from the pre-processed data. The selected feature data may be provided to the determination result providing unit 824. Here, the data obtained by the data obtaining unit 821 or the data processed by the data pre-processing unit 822 may be provided to the determination result providing unit 824 as learning data.

The data selecting unit 823 may select the feature data according to a pre-set standard. The pre-set standard may be determined by considering at least one of, for example, a data attribute, a data generation time, a data generator, data reliability, a data target, a data generation region, and a data size.

The determination result providing unit 824 may provide a determination result obtained by determining whether the patient is in a normal condition or an abnormal condition by applying the feature data to a determination model taught according to supervised learning or unsupervised learning described above. For example, the determination result providing unit 824 may provide the determination result to the controller 430 of FIG. 4. The controller 430 may control the medical image obtaining apparatus or a peripheral device, or notify a user of a condition of the patient based on the determination result.

The model updating unit 825 may control a determination model to be updated based on evaluation of the determination result provided by the determination result providing unit 824. For example, the model updating unit 825 may control the model teaching unit 814 to update the determination model by providing user feedback about the determination result provided by the determination result providing unit 824 to the model teaching unit 814.

For example, when the patient is determined to be in the abnormal condition based on the determination result provided by the determination result providing unit 824, the patient monitoring apparatus 200 may perform an operation suitable for a condition of the patient. As the operation suitable for the condition of the patient, the patient monitoring apparatus 200 may receive a user input to ignore the determination result. In this case, the model updating unit 825 may provide user feedback according to the user input to the model teaching unit 814. The user feedback may include information indicating that, for example, the determination module (e.g., classification by the determination model) is false. The model teaching unit 814 may update the determination model by using the obtained user feedback.

Alternatively, the patient monitoring apparatus 200 may receive a user input of stopping medical imaging performed on the patient or a user input of changing a medical imaging protocol. In this case, the model updating unit 825 may provide user feedback according to the user input to the model teaching unit 814. The user feedback may include, for example, information indicating that the determination result is true. Here, the user feedback may include information about the changed medical imaging protocol. The model teaching unit 814 may update the determination model by using the obtained user feedback.

At least one of the data obtaining unit 821, the data pre-processing unit 822, the data selecting unit 823, the determination result providing unit 824, and the model updating unit 825 of the determining unit 820 may be manufactured in at least one hardware chip form and mounted on an electronic device. For example, at least one of the data obtaining unit 821, the data pre-processing unit 822, the data selecting unit 823, the determination result providing unit 824, and the model updating unit 825 may be manufactured in an exclusive hardware chip form for Al, or may be manufactured as part of an existing general-purpose processor or graphic-exclusive processor and included in any electronic device.

Also, the data obtaining unit 821, the data pre-processing unit 822, the data selecting unit 823, the determination result providing unit 824, and the model updating unit 825 may be included in one electronic device or on different electronic devices. For example, some of the data obtaining unit 821, the data pre-processing unit 822, the data selecting unit 823, the determination result providing unit 824, and the model updating unit 825 may be included in the patient monitoring apparatus 200, and the remainder may be included in a server.

Also, at least one of the data obtaining unit 821, the data pre-processing unit 822, the data selecting unit 823, the determination result providing unit 824, and the model updating unit 825 may be implemented in a software module (or a program module including an instruction). Here, the software module may be stored in a non-transitory computer-readable recording medium. The software module may be provided by an OS or a certain application. Alternatively, a part of the software module may be provided by an OS and the remainder of the software module may be provided by a certain application.

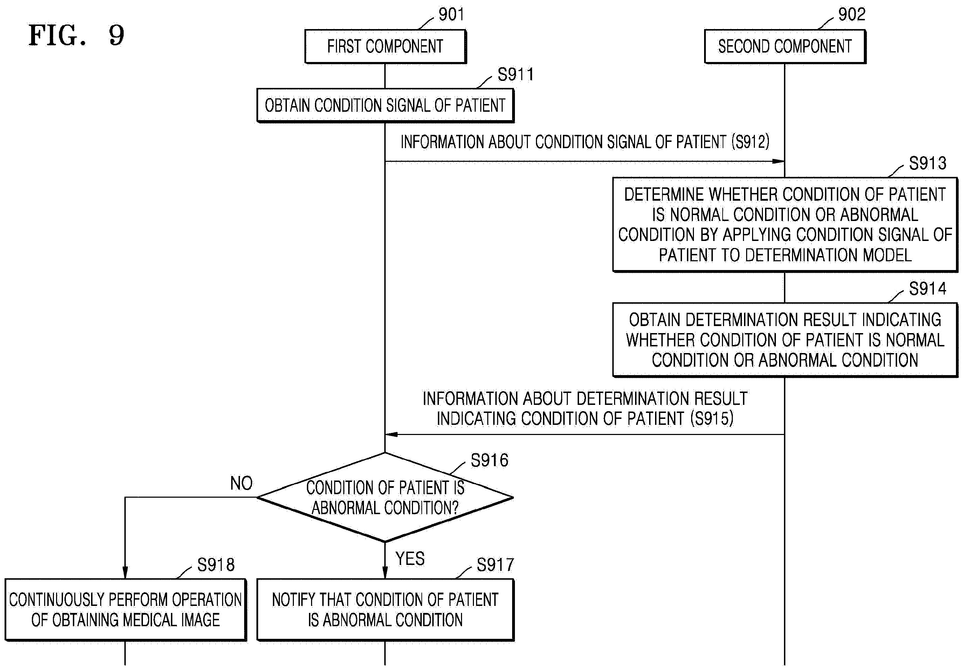

FIG. 9 is a flowchart of a system using a determination model, according to an embodiment.

In FIG. 9, the system may include a first component 901 and a second component 902. Here, the first component 901 may be the patient monitoring apparatus 200 and the second component 902 may be a server providing a determination result of determining a normal condition or an abnormal condition of a patient. Alternatively, the first component 901 may be a general-purpose processor and the second component 902 may be an Al-exclusive processor. Alternatively, the first component 901 may be at least one application and the second component 902 may be an OS. In other words, the second component 902 may be a component that is more integrated, is more dedicated, has smaller delay, has better performance, or has more resources than the first component 901, and may process many operations required while generating, updating, or applying a determination model, more quickly and effectively than the first component 901.

At this time, an interface for transmitting/receiving data between the first and second components 901 and 902 may be defined.

For example, an application program interface (API) having learning data to be used to teach the determination model as a factor value (a parameter value or a transmission value) may be defined. Alternatively, an API having feature data to be used to determine the determination model as a factor value may be defined. An API may be defined as a group of sub-routines or functions which may be called by one protocol (for example, a protocol defined by the patient monitoring apparatus 200) to perform a process of another protocol (for example, a protocol defined by a server). In other words, an environment in which one protocol may perform an operation of another protocol may be provided through an API.

In operation S911 of FIG. 9, the first component 901 may obtain a condition signal of a patient. For example, the first component 901 may obtain the condition signal of the patient from at least one sensor detecting a condition of the patient in an operation of imaging the patient.

Then, the second component 902 may determine whether the condition of the patient is the normal condition or the abnormal condition by using a model that is set to determine the abnormal condition of the patient, based on the obtained condition signal of the patient.

In detail, in operation S912, the first component 901 may transmit information about the obtained condition signal of the patient to the second component 902. For example, the first component 901 may apply the condition signal of the patient as a factor value of an API function provided to use a determination model. The API function may transmit the condition signal as feature data to be applied to the determination model, to the second component 902. Here, the information about the condition signal of the patient may include communication data that is amended, supplemented, or has additional headers according to a communication format.

In operation S913, the second component 902 may determine whether the condition of the patient is the normal condition or the abnormal condition by applying the received condition signal of the patient to the determination model. For example, the second component 902 may determine whether the condition of the patient is the normal condition or the abnormal condition by applying the condition signal of the patient to the determination model stored in a memory of the second component 902 or the determination model connected to the second component 902 via wired communication or wireless communication.

In operation S914, the second component 902 may obtain a determination result indicating whether the condition of the patient is the normal condition or the abnormal condition.

In operation S915, the second component 902 may transmit information about the determination result to the first component 901.

In operation S916, the first component 901 may determine whether the condition of the patient is the abnormal condition based on the determination result.

In operation S917, when the condition of the patient is determined to be the abnormal condition, the first component 901 may notify a user that the condition of the patient is the abnormal condition. In operation S918, when the condition of the patient is determined to be the normal condition, the first component 901 may continuously perform an operation of obtaining a medical image.

Then, the first component 901 may control a medical image obtaining apparatus based on a user input according to the determination result. Corresponding operations have been described with reference to operations S670 to S690 of FIG. 6, and thus repetitive descriptions are not provided.

The exemplary embodiments of the disclosure can be written as computer programs and can be implemented in general-use digital computers that execute the programs using a computer-readable recording medium. Also, a structure of data used in the above embodiments may be recorded on a computer-readable recording medium via various methods. Also, an embodiment of the disclosure may also be realized in a form of a computer-readable recording medium, such as a program module executed by a computer. For example, when software modules are involved, these software modules may be stored as program instructions or computer-readable codes executable on the processor on a computer-readable media.

A computer-readable recording medium may be an arbitrary available medium accessible by a computer, and examples thereof include all volatile and non-volatile media and separable and non-separable media. Examples of the computer-readable recording medium may include a magnetic storage medium (e.g., a read-only memory (ROM), floppy disks, hard disks, etc.), an optical recording medium (e.g., compact disc (CD)-ROMs, or digital versatile discs (DVDs)), etc., but are not limited thereto. Also, the computer-readable recording medium may include a computer storage medium and a communication medium.

The computer-readable recording medium may also be distributed over network-coupled computer systems so that the computer-readable code is stored and executed in a distributed fashion.

The computer-readable recording medium may be a non-transitory storage medium. Here, the term 'non-transitory' means that a storage medium does not include a signal and is tangible, and does not distinguish whether data is stored in the storage medium semi-permanently or temporarily.

Also, the embodiments may be provided by being included in a computer program product. The computer program product is a product that may be traded between a seller and a purchaser.

The computer program product may include a software program and a computer-readable recording medium having recorded thereon the software program. For example, the computer program product may include a product (for example, a downloadable application) in a form of a software program electronically distributed through an electronic market (for example, Google Play or Appstore) or a manufacturer of a patient monitoring apparatus. For electronic distribution, at least a part of the software program may be stored in a storage medium or may be temporarily generated. In this case, the storage medium may be a storage medium of a server of the manufacturer, a server of the electronic market, or a relay server that temporarily stores the software program.

For the sake of brevity, conventional electronics, control systems, software development and other functional aspects of the systems (and components of the individual operating components of the systems) may not be described in detail.

Terms such as "unit" and "module" indicate a unit for processing at least one function or operation, wherein the unit and the block may be embodied as hardware or software or embodied by combining hardware and software. The "unit" or "module" may be formed so as to be in an addressable storage medium, or may be formed so as to operate one or more processors. For example, the term "unit" or "module" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables.

At least one of the components, elements, modules or units represented by a block as illustrated in the drawings may be embodied as various numbers of hardware, software and/or firmware structures that execute respective functions described above, according to an exemplary embodiment. For example, at least one of these components, elements or units may use a direct circuit structure, such as a memory, a processor, a logic circuit, a look-up table, etc. that may execute the respective functions through controls of one or more microprocessors or other control apparatuses. Also, at least one of these components, elements or units may be specifically embodied by a module, a program, or a part of code, which contains one or more executable instructions for performing specified logic functions, and executed by one or more microprocessors or other control apparatuses. Also, at least one of these components, elements or units may further include or implemented by a processor such as a central processing unit (CPU) that performs the respective functions, a microprocessor, or the like. Two or more of these components, elements or units may be combined into one single component, element or unit which performs all operations or functions of the combined two or more components, elements of units. Also, at least part of functions of at least one of these components, elements or units may be performed by another of these components, element or units. Further, although a bus is not illustrated in the above block diagrams, communication between the components, elements or units may be performed through the bus. Functional aspects of the above exemplary embodiments may be implemented in algorithms that execute on one or more processors. Furthermore, the components, elements or units represented by a block or processing steps may employ any number of related art techniques for electronics configuration, signal processing and/or control, data processing and the like.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the spirit and scope as defined by the following claims.

## Claims

1. A method for monitoring a condition of an object, comprising:
obtaining a condition signal of the object in an operation of imaging the object to obtain a medical image of the object;
determining whether the condition of the object is an abnormal condition based on the condition signal, by using a model that is set to determine the abnormal condition of the object; and
in response to determining that the condition of the object is the abnormal condition, performing an operation corresponding to the abnormal condition of the object,
wherein the model is generated by learning data obtained in relation to the object by using an artificial intelligence (Al) algorithm.

2. The method of claim 1, wherein the condition signal of the object is obtained from at least one sensor or obtained by analyzing the medical image of the object.

3. The method of claim 1, wherein the model is generated by further learning data obtained in relation to other objects.

4. The method of claim 1, wherein the model is generated by learning a pre-imaging condition signal of the object before the medical image of the object is obtained.

5. The method of claim 1, wherein the model is generated by learning standard information indicating whether the data obtained in relation to the object is data obtained in a normal condition of the object or data obtained in the abnormal condition of the object.

6. The method of claim 1, wherein the condition signal comprises at least one of an image signal of the object, a voice signal of the object, an electrocardiogram, a heart rate, blood pressure, oxygen saturation, a respiration state, a respiration rate, a temperature of the object, and a temperature around the object.

7. The method of claim 1, wherein the determining whether the condition of the object is the abnormal condition comprises determining whether the condition of the object is the abnormal condition by using the model, based on the condition signal and a protocol being performed to obtain the medical image.

8. The method of claim 1, wherein the performing the operation corresponding to the abnormal condition of the object comprises performing at least one of changing a protocol for obtaining the medical image, stopping an operation of obtaining the medical image, restarting the operation of obtaining the medical image, attempting to communicate with the object, notifying that the condition of the object is abnormal, and removing the object from a medical image obtaining apparatus.

9. The method of claim 1, further comprising, in response to determining that the condition of the object is a normal condition, continuously performing an operation of obtaining the medical image,
wherein the performing the operation corresponding to the abnormal condition of the object comprises:
notifying a user that the condition of the object is the abnormal condition; and
in response to receiving a user input, continuously performing the operation of obtaining the medical image.

10. The method of claim 9, further comprising:
obtaining user feedback according to the user input; and
controlling the model to be updated by using the user feedback.

11. An apparatus for monitoring a condition of an object, comprising:
a model generated by learning data obtained in relation to the object by using an artificial intelligence (AI) algorithm, and set to determine an abnormal condition of the object; and
at least one processor configured to determine, based on a condition signal of the object obtained in an operation of imaging the object to obtain a medical image of the object, whether the condition of the object is the abnormal condition, by using the model which is set to determine the abnormal condition of the object, and in response to determining that the condition of the object is the abnormal condition, perform an operation corresponding to the abnormal condition of the object.

12. The apparatus of claim 11, wherein the condition signal of the object is obtained from at least one sensor or by analyzing the medical image of the object.

13. The apparatus of claim 11, wherein the model is generated by further learning data obtained in relation to other objects.

14. The apparatus of claim 11, wherein the model is generated by learning a pre-imaging condition signal of the object before the medical image of the object is obtained.

15. A system for monitoring a condition of an object, comprising:
an apparatus configured to determine, based on a condition signal of the object obtained in an operation of imaging the object to obtain a medical image of the object, whether the condition of the object is an abnormal condition by using a model which is set to determine the abnormal condition of the object, and in response to determining that the condition of the object to be the abnormal condition, perform an operation corresponding to the abnormal condition of the object; and
a medical image obtaining apparatus configured to perform an operation of obtaining the medical image of the object in response to determining that the condition of the object is a normal condition,
wherein the model is generated by learning data obtained in relation to the object by using an artificial intelligence (Al) algorithm.
